# EUROPEAN PATENT APPLICATION

(11) **EP 2 719 382 A1**
(43) Date of publication of application: **16.04.2014**
(21) Application number: 12188329.2
(22) Date of filing: 12.10.2012
(51) Int. Cl.: A61K 31/202, A61K 31/232, A61P 1/16

(54) **Ethyl Eicosapentanoate and Pharmaceutical Compositions Comprising Ethyl Eicosapentanoate as an Active Ingredient for Use in the Treatment of Non-Alcoholic Steatohepatitis**

(71) Applicant: MOCHIDA PHARMACEUTICAL CO., LTD., Shinjuku-ku Tokyo 160-8515 (JP)
(72) Inventor: Mizuguchi, Kiyoshi, Shinjuku-ku, Tokyo 160-8515 (JP); Harada, Tsuyoshi, Shinjuku-ku, Tokyo 160-8515 (JP); Osada, Atsushi, Shinjuku-ku, Tokyo 160-8515 (JP); Kawano, Hiroyuki, Shinjuku-ku, Tokyo 160-8515 (JP); Ichioka, Masayuki, Shinjuku-ku, Tokyo 160-8515 (JP)
(74) Representative: Grünecker, Kinkeldey, Stockmair & Schwanhäusser

(57) **Abstract**

Ethyl eicosapentanoate and compositions comprising this compound as an active ingredient for use in the treatment or alleviation of symptoms of non-alcoholic steatohepatitis (NASH) are disclosed.

## Description

### TECHNICAL FIELD

The present invention relates to ethyl eicosapentanoate and compositions comprising this compound as an active ingredient for use in the treatment of non-alcoholic steatohepatitis (NASH), and for alleviation of the symptoms associated with NASH.

### BACKGROUND ART

It is known that heavy alcohol use can lead to liver complications, including alcoholic hepatitis, which is often characterized by fatty liver and inflammation. Alcoholic hepatitis can ultimately lead to cirrhosis of the liver (scarring) and hardening of the liver tissue.

Individuals who do not consume excessive amounts of alcohol can also develop liver disease complications. Non-alcoholic fatty liver disease (NAFLD) is understood to encompass a variety of liver diseases, including steatosis (simple fatty liver), non-alcoholic steatohepatitis (NASH) and advanced scarring of the liver (cirrhosis).

The pathogenesis of NASH is poorly understood but it is probably multifactorial. Insulin resistance has emerged as a condition common to all forms of NASH and it is probably primarily important in producing steatosis as it is found in NAFLD as well as in NASH. However, other factors are also required for progression to hepatitis and such factors include oxidative stress damaging membrane lipids and possibly leptin. These factors may cause production of proinflammatory cytokines, stellate cell activation, fibrosis and hepatic cell damage.

Clinically, NASH is usually asymptomatic and is therefore often an incidental diagnosis made from liver function tests at health checks or in the investigation of other disease. Care is needed in excluding alcohol abuse. It may account for up to 90% of isolated increases of plasma transaminases of otherwise unknown cause. Fatigue and malaise have been attributed to NASH, and a few patients have abdominal pain reproduced by pressure over an enlarged liver. Hepatomegaly on palpation is found in about three-quarters of patients but other features of cirrhosis are rare. It is now suspected that there is an even distribution of NASH among men and women. Obesity, diabetes mellitus, hypertension and hyperlipidemia are common associated features. A number of drugs such as corticosteroids, synthetic estrogens, tamoxifen, perhexiline and amiodarone have also been associated with NASH.

Liver function tests show that plasma transaminase activity may be increased usually about 2-4-fold (alanine amino-transferase usually more than aspartate amino-transferase), plasma alkaline phosphatase is mildly increased in about one-third, and plasma albumin is normal. The plasma ferritin and transferrin saturation may be increased.

NASH has traditionally been diagnosed by means of a liver biopsy to characterize the liver histology, particularly with respect to the characteristics of inflammation, fibrosis and steatosis (fat accumulation). NASH then generally refers to clinical findings based upon the liver biopsy of a patient with steatohepatitis, combined with the absence of significant alcohol consumption (Neuschwander-Tetri, B.A. and S.H. Caldwell (2003) Hepatology 37(5): 1202-1209). In NASH, fat accumulation is seen in varying degrees of inflammation (hepatitis) and scarring (fibrosis). Patients having NASH are also often characterized by abnormal levels of liver enzymes, such as aspartate aminotransferase (AST) and alanine aminotransferase (ALT). However, a clinical diagnosis of NASH still depends upon a liver biopsy to assess the histologic characteristics of the patient's liver, such that histological examination of liver biopsy tissue is often characterized as the "gold-standard" technique for the assessment of liver fibrosis (*Neuschwander-Tetri, ibid*)*.*

Eicosapentaenoic acid (EPA) is a known omega-3 polyunsaturated, long-chain fatty acid. Omega-3 fatty acids are known as components of oils, such as fish oil, and a variety of commercial products are promoted as containing omega-3 fatty acids, or their esters, derivatives, conjugates and the like. Eicosapentaenoic acid (EPA) is also *per se* known in its ethyl ester form, ethyl eicosapentanoate (ethyl all-cis-5,8,11,14,17-eicosapentaenoate; EPA-E).

### SUMMARY OF THE INVENTION

It is the problem of the present invention to provide a compound for use in the treatment or alleviation of symptoms of non-alcoholic steatohepatitis.

The above problem is solved by ethyl eicosapentanoate for use in the treatment or alleviation of symptoms of non-alcoholic steatohepatitis, wherein said ethyl eicosapentanoate is administered in a dose of between 300 and 4000 mg per day.

The present invention also provides pharmaceutical compositions comprising the compound for use according to the present invention as an active ingredient.

The following embodiments are representative of the present invention:
1. Ethyl eicosapentanoate for use in the treatment or alleviation of symptoms of non-alcoholic steatohepatitis,
   wherein said ethyl eicosapentanoate is administered in a dose of between 300 and 4000 mg per day.
2. Ethyl eicosapentanoate for use according to embodiment 1,
   wherein said ethyl eicosapentanoate is administered in a dose of between 900 and 3600 mg per day.
3. Ethyl eicosapentanoate for use according to embodiment 1 or 2, wherein said dose is between 1800 and 2700 mg per day.
4. Ethyl eicosapentanoate for use according to any of embodiments 1 to 3, wherein said ethyl eicosapentanoate is administered once to three times per day in dosage amounts of 600 mg or 900 mg.
5. Ethyl eicosapentanoate for use according to any of embodiments 1 to 4, wherein said ethyl eicosapentanoate is administered for a period of between 3 and 24 months.
6. Ethyl eicosapentanoate for use according to embodiment 5, wherein said ethyl eicosapentanoate is administered for a period of between 6 and 12 months.
7. A pharmaceutical composition for use in the treatment or alleviation of symptoms of non-alcoholic steatohepatitis,
   comprising ethyl eicosapentanoate as an active ingredient, wherein said ethyl eicosapentanoate is administered in a dose of between 300 and 4000 mg per day.
8. The pharmaceutical composition for use according to embodiment 7, wherein said ethyl eicosapentanoate is administered in a dose of between 900 and 3600 mg per day.
9. The composition for use according to embodiment 7 or 8, wherein said composition further comprises one or more fatty acid.
10. The composition for use according to embodiment 9, wherein said fatty acid is Docosahexaenoic ethyl ester.
11. The composition for use according to embodiment 9 or 10, wherein the weight ratio between ethyl eicosapentanoate and the fatty acid is 0.8 or more.
12. The composition for use according to any of embodiments 9 to 11, wherein the daily dose in terms of total content of ethyl eicosapentanoate and said fatty acid is 0.3 to 10.0 g/day.

In another embodiment of the invention, the ethyl icosapentanoate for use in treatment or alleviation of symptoms NASH, wherein;
a subject is identified having NASH characterized by baseline levels of ALT of between 5 to 300 U/L and at least one criteria selected from the group consisting of NAS score of 4 or more than 4, a steatosis score of 1 or more than 1, a lobular inflammation score of 1 or more than 1 and either (i) a fibrosis stage of at least 1a or (ii) ballooning, and at least one or any combination of two or more of the pretreatment baseline of the items mentioned in Tables 1 and 2;
a baseline level in blood or physical condition prior to treatment in the subject is determined,;
an effective amount of ethyl icosapentanoate is administered to the subject; and the NAS score in the subject (i) to a composite score of 3 or less than 3, or (ii)
by 2 or more than 2 across at least two of the NAS components, together with no worsening of the fibrosis stage score,
optionally improving at least one selected from the items mentioned in Tables 1 and 2 is improved.

In another embodiment of the invention, the ethyl icosapentanoate for use in treatment or alleviation of symptoms NASH, the subject is taking at least one drug selected from the group consisting of lipid-lowering drugs, HMG-CoA reductase inhibitors (stains), fibrates, probucol, ezetimibe, ursodiol (UDCA), taurine, betaine, N-acetylcysteine, s-adenosylmethionine (SAM-e), milk thistle, anti-TUMOR NECROSIS FACTOR (TNF) therapies, probiotics, anti-diabetic medications, biguanides (metformin), insulin, sulfonylureas, alpha-glucosidase inhibitors (acarbose), dipeptidyl-peptidase 4 inhibitors (sitagliptin, saxagliptin, alogliptin, vildagliptin, linagliptin, etc.), phenylalanine derivatives (nateglinide, repaglinide), anti-platelet therapy, anti-thrombotic agents, Glucagon-like peptide-1(GLP-1) receptor agonists (liraglutide, exenatide, taspoglutide, etc.), PDE-4 inhibitor, angiotensin II-1 type receptor antagonist (ARB: losartan, etc.), polyenephosphatidylcholine, antioxidant (vitamine E, vitamin C, nicotinic acid tocopherol,etc.), and pentoxifylline.

In another embodiment of the invention, ethyl icosapentanoate for use in the treatment or alleviation of symptoms of NASH wherein,
a subject is identifyed having NASH characterized by baseline levels of ALT of between 5 to 300 U/L and at least one criteria selected from the group consisting of NAS score of 4 or more than 4, a steatosis score of 1 or more than 1, a lobular inflammation score of 1 or more than 1 and either (i) a fibrosis stage of at least 1a or (ii) ballooning, and at least one or any combination of two or more of the pretreatment baseline of the items mentioned in Tables 1 and 2;
a baseline level in blood or physical condition prior to treatment in the subject is determined;
an effective amount of ethyl icosapentanoate administering to the subject in combination with at least one drug selected from the group consisting of lipid-lowering drugs, HMG-CoA reductase inhibitors (stains), fibrates, probucol, ezetimibe, ursodiol (UDCA), taurine, betaine, N-acetylcysteine, s-adenosylmethionine (SAM-e), milk thistle, anti-TNF therapies, probiotics, anti-diabetic medications: biguanides (metformin), insulin, sulfonylureas, alpha-glucosidase inhibitors (acarbose), dipeptidyl-peptidase 4 inhibitors (sitagliptin, saxagliptin, alogliptin, vildagliptin, linagliptin, etc.), phenylalanine derivatives (nateglinide, repaglinide), anti-platelet therapy, anti-thrombotic agents, Glucagon-like peptide-1(GLP-1) receptor agonists (liraglutide, exenatide, taspoglutide, etc.), PDE-4 inhibitor, angiotensin II-1 type receptor antagonist (ARB: losartan, etc.), polyenephosphatidylcholine, antioxidant (vitamine E, vitamin C, nicotinic acid tocopherol,etc.), and pentoxifylline; and
the NAS score in the subject is improved (i) to a composite score of 3 or less than 3, or (ii) by ≥ 2 across at least two of the NAS components, together with no worsening of the fibrosis stage score, optionally improving at least one of items mentioned in Tables 1 and 2.

In another embodiment of the invention, the ethyl icosapentanoate for use in reducing steatosis, liver lobular inflammation, ballooning and/or liver fibrosis in a subject in need thereof, wherein;
a baseline level in blood or physical condition prior to treatment in the subject having at least one item or any combination of two or more items selected from the pretreatment baseline of the items mentioned in Tables 1 and 2 is determined;
an effective amount of ethyl icosapentanoate (EPA-E) is administered to the subject;
at least one condition selected from the group consisting of the steatosis, lobular inflammation, ballooning and liver fibrosis condition of said subject without worsening said fibrosis stage score is improved; and
said subject exhibits the described changes in at least one of items mentioned in Tables 1 and 2 as compared to a baseline pre-treatment level of the item.

In another embodiment of the invention, the ethyl icosapentanoate for use in reducing steatosis, liver lobular inflammation, ballooning and/or liver fibrosis in a subject in need thereof, the subject is taking at least one drug selected from the group consisting of lipid-lowering drugs, HMG-CoA reductase inhibitors (stains), fibrates, probucol, ezetimibe, ursodiol (UDCA), taurine, betaine, N-acetylcysteine, s-adenosylmethionine (SAM-e), milk thistle, anti-TNF therapies, probiotics, anti-diabetic medications: biguanides (metformin), insulin, sulfonylureas, alpha-glucosidase inhibitors (acarbose), dipeptidyl-peptidase 4 inhibitors (sitagliptin, saxagliptin, alogliptin, vildagliptin, linagliptin, etc.), phenylalanine derivatives (nateglinide, repaglinide), anti-platelet therapy ,anti-thrombotic agents, Glucagon-like peptide-1(GLP-1) receptor agonists (liraglutide, exenatide, taspoglutide, etc.), PDE-4 inhibitor, angiotensin II-1 type receptor antagonist (ARB: losartan, etc.), polyenephosphatidylcholine, antioxidant (vitamine E, vitamin C, nicotinic acid tocopherol,etc.), and pentoxifylline.

In another embodiment of the invention, the ethyl icosapentanoate for use in the treatment or alleviation of symptoms of NASH, wherein the subject is possible or definite NASH, wherein an effective amount of ethyl icosapentate is administered to a subject, wherein a baseline level in blood or physical condition prior to treatment in the subject of at least one item or any combination of two or more items selected from the items mentioned in Tables 1 and 2 is determined.

In another embodiment of the invention, the ethyl icosapentanoate for use in the treatment or alleviation of symptoms of NASH, wherein the subject is possible or definite NASH and the subject is taking at least one drug selected from the group consisting of lipid-lowering drugs, HMG-CoA reductase inhibitors (stains), fibrates, probucol, ezetimibe, ursodiol (UDCA), taurine, betaine, N-acetylcysteine, s-adenosylmethionine (SAM-e), milk thistle, anti-TNF therapies, probiotics, anti-diabetic medications: biguanides (metformin), insulin, sulfonylureas, alpha-glucosidase inhibitors (acarbose), dipeptidyl-peptidase 4 inhibitors (sitagliptin, saxagliptin, alogliptin, vildagliptin, linagliptin, etc.), phenylalanine derivatives (nateglinide, repaglinide), anti-platelet therapy ,anti-thrombotic agents, Glucagon-like peptide-1(GLP-1) receptor agonists (liraglutide, exenatide, taspoglutide, etc.), PDE-4 inhibitor, angiotensin II-1 type receptor antagonist (ARB: losartan, etc.), polyenephosphatidylcholine, antioxidant (vitamine E, vitamin C, nicotinic acid tocopherol,etc.), and pentoxifylline.

In another embodiment of the invention, the ethyl icosapentanoate for use in the treatment or alleviation of symptoms of NASH wherein an effective amount of ethyl icosapentate is administered to a subject being possible or definite NASH, and exhibits the described changes of after dosing value in said at least one item selected from the items mentioned in Tables 1 and 2 as compared to a baseline pre-treatment level thereof.

In another embodiment of the invention, the ethyl icosapentanoate for use in the treatment or alleviation of symptoms of NASH wherein the subject is possible or definite NASH and the subject is taking at least one drug selected from the group consisting of lipid-lowering drugs, HMG-CoA reductase inhibitors (stains), fibrates, probucol, ezetimibe, ursodiol (UDCA), taurine, betaine, N-acetylcysteine, s-adenosylmethionine (SAM-e), milk thistle, anti-TNF therapies, probiotics, anti-diabetic medications: biguanides (metformin), insulin, sulfonylureas, alpha-glucosidase inhibitors (acarbose), dipeptidyl-peptidase 4 inhibitors (sitagliptin, saxagliptin, alogliptin, vildagliptin, linagliptin, etc.), phenylalanine derivatives (nateglinide, repaglinide), anti-platelet therapy ,anti-thrombotic agents, Glucagon-like peptide-1(GLP-1) receptor agonists (liraglutide, exenatide, taspoglutide, etc.), PDE-4 inhibitor, angiotensin II-1 type receptor antagonist (ARB: losartan, etc.), polyenephosphatidylcholine, antioxidant (vitamine E, vitamin C, nicotinic acid tocopherol,etc.), and pentoxifylline.

In another embodiment of the invention, the ethyl icosapentanoate for use in the treatment or alleviation of symptoms of NASH wherein an effective amount of ethyl icosapentanoate is administered to a subject, wherein the subject is taking at least one drug selected from the group consisting of lipid-lowering drugs, HMG-CoA reductase inhibitors (stains), fibrates, probucol, ezetimibe, ursodiol (UDCA), taurine, betaine, N-acetylcysteine, s-adenosylmethionine (SAM-e), milk thistle, anti-TNF therapies, probiotics, anti-diabetic medications: biguanides (metformin), insulin, sulfonylureas, alpha-glucosidase inhibitors (acarbose), dipeptidyl-peptidase 4 inhibitors (sitagliptin, saxagliptin, alogliptin, vildagliptin, linagliptin, etc.), phenylalanine derivatives (nateglinide, repaglinide), anti-platelet therapy ,anti-thrombotic agents, Glucagon-like peptide-1(GLP-1) receptor agonists (liraglutide, exenatide, taspoglutide, etc.), PDE-4 inhibitor, angiotensin II-1 type receptor antagonist (ARB: losartan, etc.), polyenephosphatidylcholine, antioxidant (vitamine E, vitamin C, nicotinic acid tocopherol,etc.), and pentoxifylline.

In another embodiment of the invention, the ethyl icosapentanoate for use in the treatment or alleviation of symptoms of NASH wherein an effective amount of ethyl icosapentanoate is administered to a subject, wherein the subject is taking at least one lipid-lowering drug.

In another embodiment of the invention, the ethyl icosapentanoate for use in the treatment or alleviation of symptoms of NASH wherein an effective amount of ethyl icosapentanoate is administered to a subject, wherein the subject is taking an HMG-CoA reductase inhibitor (statins; pravastatin sodium, simvastatin, pitavastatin calcium, atorvastatin calcium hydrate, rosuvastatin calcium, etc.).

In another embodiment of the invention, the ethyl icosapentanoate for use in the treatment or alleviation of symptoms of NASH wherein an effective amount of ethyl icosapentanoate is administered to a subject, wherein the subject is taking a Glucagon-like peptide-1 (GLP-1) receptor agonist (liraglutide, exenatide, taspoglutide, etc.).

In another embodiment of the invention, the ethyl icosapentanoate for use in the treatment or alleviation of symptoms of NASH wherein an effective amount of ethyl icosapentanoate is administered to a subject in combination with at least one drug selected from the group consisting of lipid-lowering drugs, HMG-CoA reductase inhibitors (stains), fibrates, probucol, ezetimibe, ursodiol (UDCA), taurine, betaine, N-acetylcysteine, s-adenosylmethionine (SAM-e), milk thistle, anti-TNF therapies, probiotics, anti-diabetic medications: biguanides (metformin), insulin, sulfonylureas, alpha-glucosidase inhibitors (acarbose), dipeptidyl-peptidase 4 inhibitors (sitagliptin, saxagliptin, alogliptin, vildagliptin, linagliptin, etc.), phenylalanine derivatives (nateglinide, repaglinide), anti-platelet therapy ,anti-thrombotic agents, Glucagon-like peptide-1(GLP-1) receptor agonists (liraglutide, exenatide, taspoglutide, etc.), PDE-4 inhibitor, angiotensin II-1 type receptor antagonist (ARB: losartan, etc.), polyenephosphatidylcholine, antioxidant (vitamine E, vitamin C, nicotinic acid tocopherol,etc.), and pentoxifylline.

In another embodiment of the invention, the ethyl icosapentanoate for use in the treatment or alleviation of symptoms of NASH wherein an effective amount of ethyl icosapentanoate is administered to a subject, wherein the subject is not diabetic.

In another embodiment of the invention, the ethyl icosapentanoate for use in the treatment or alleviation of symptoms of NASH wherein an effective amount of ethyl icosapentanoate is administered to a subject, wherein the subject has diabetes.

In another embodiment of the invention, the ethyl icosapentanoate for use in the treatment or alleviation of symptoms of NASH wherein an effective amount of ethyl icosapentanoate is administered to a subject, wherein the subject has impaired glucose tolerance.

In another embodiment of the invention, the ethyl icosapentanoate for use in the treatment or alleviation of symptoms of NASH wherein an effective amount of ethyl icosapentanoate is administered to a subject, wherein the subject has metabolic syndrome.

### DETAILED DESCRIPTION OF THE INVENTION

The EPA-E for use according to the present invention is administered in a dose of between 300 and 4000 mg per day, alternatively 0.6-6 g per day, alternatively 0.9-3.6 g per day. Preferably the dose of EPA-E is 1-2.6 g per day (such as 1-2 g per day), or more preferably 1.5-2.5 g per day (such as 1.7-2.2 g per day). The dose of EPA-E is, for example, 600 mg per day, 1000 mg per day, 1500 mg per day, 1800 mg per day, 2000 mg per day, 2200 mg per day, 2600 mg per day, or 2700 mg per day.

The pharmaceutical compositions for use according to the present invention comprise EPA-E as an active ingredient. The compositions of the present invention may comprise, in addition to EPA-E, other fatty acids and/or derivatives thereof. The EPA-E content among the total fatty acid content of the compositions of the present invention is not particularly limited as long as the composition contains EPA-E as its effective component. However, pure EPA-E is preferably used. For example, the compositions of the present invention have a proportion of EPA-E of preferably 40% by weight or more, more preferably 90% by weight or more, and still more preferably 96.5% by weight or more based on the total amount of the fatty acids and/or their derivatives in the composition. EPA-E can be administered to patients in a highly purified form, including the product known as Epadel® (Mochida Pharmaceutical Co., Ltd., Tokyo Japan).

The pharmaceutical compositions for use according to the present invention are administered to a subject or patient in a dose of between 300 and 4000 mg per day (such as between 400 and 2600 mg per day), alternatively 0.6-6 g per day, alternatively 0.9-3.6 g per day. Preferably the dose of EPA-E is 1-2.6 g per day (such as 1-2 g per day), or more preferably 1.5-2.5 g per day (such as 1.7-2.2 g per day). The dose of EPA-E is, for example, 600 mg per day, 1000 mg per day, 1500 mg per day, 1800 mg per day, 2000 mg per day, 2200 mg per day, 2500 mg per day, 2600 mg per day or 2700 mg per day.

In embodiments, the compositions for use according to the present invention may contain other fatty acids besides EPA-E. For example, they may contain any omega-3 unsaturated fatty acid, especially DHA-E (Docosahexaenoic ethyl ester). The ratio of EPA-E/DHA-E in the composition, the content of EPA-E and DHA-E in the total fatty acids and administration amount of EPA-E and DHA-E are not limited but the ratio is preferably 0.8 or more, more preferably 1.0 or more, still more preferably 1.2 or more. The composition is preferably highly purified; for example, the proportion of EPA-E+DHA-E among the fatty acids and their derivatives in the composition is preferably 40% by weight or more, more preferably 80% by weight or more, and still more preferably 90% or more. The daily amount in terms of EPA-E+DHA-E is typically 0.3 to 10.0 g/day, preferably 0.5 to 6.0 g/day, and still more preferably 1.0 to 4.0 g/day. The low content of other long chain saturated fatty acids is preferred, and among the long chain unsaturated fatty acids, the content of omega-6 fatty acids, and in particular, the content of arachidonic acid is preferably as low as less than 2% by weight, and more preferably less than 1% by weight.

In another embodiment, the composition for use according to the present invention contains a fatty acid. For examples, the fatty acid is preferably (C15:1) ω5cis Pentadecenoic, (C20:2) ω6 Eicosadienoic, (C16:0) Palmitic, (C20:3)ω3 Eicosatrienoic(ETE), (C16:1) ω7c Palmitoleic, (C20:3) ω6 Dihomo-g-linolenic (DGLA), (C17:0) Heptadecanoic, (C20:4) ω6 Arachidonic (AA), (C17:1) ω7 Heptadecenoic, (C20:5) ω3 Eicosapentaenoic (EPA), (C18:0) Stearic, (C21:0) Heneicosanoic, (C18:1) ω9c Oleic, (C22:0) Behenic, (C18:1) ω9t Elaidic, (C22:1) ω9 Erucic, (C18:2) ω6 Linoleic (LA), (C22:2) ω6 Docosadienoic, (C18:2) ω6t Linolelaidic, (C22:6) ω3 Docosahexaenoic (DHA), (C18:3) ω3, alpha-Linolenic (ALA), (C22:5) ω3 Docosapentaenoic (DPA), (C18:3) ω6 gamma-Linolenic (GLA), (C23:0) Tricosanoic, (C20:0) Arachidic, (C24:0) Lignoceric, (C20:1) ω9 Eicosenoic, (C24:1) ω9c Nervonic acid and combinations of two, three or more types of these fatty acids. The weight ratio between the content of EPA-E and the fatty acid(s) in the composition is preferably 0.8 or more, more preferably 1.0 or more, still more preferably 1.2 or more. The proportion of EPA-E and the fatty acid based on the total content of fatty acids and their derivatives present in the composition is preferably 40% by weight or more, more preferably 80% by weight or more, and still more preferably 90% or more. The daily amount in terms of content of EPA-E and the above fatty acid(s) is typically 0.3 to 10.0 g/day, preferably 0.5 to 6.0 g/day, and still more preferably 1.0 to 4.0 g/day.

The compound or the pharmaceutical compositions for use according to the invention can be administered to a patient in need of treatment of NASH for 3, 6 or 9 months, or for 1 year or more and can be administered in one, two or three dosages per day, or other multiple doses per day including 1 to about 10, 1 to 8, 1 to 6, 1 to 4 or 1 to 2 dosage units per day as appropriate for patient therapy. The term "dose unit" and "dosage unit" herein refer to a portion of a pharmaceutical composition that contains an amount of EPA-E for a single administration to a subject.

While meal affects the absorption of the EPA-E, and the administration of the EPA-E is preferably conducted during the meal or after the meal, and more preferably immediately after the meal (within 30 minutes after the meal).The self-emulsifying composition has excellent absorption under fasting, and therefore, it exhibits the intended effects even when administered at a timing other than during, after, or immediately after the meal.

In the present invention the term "patient in need of treatment of NASH" refers to a human subject/patient who has a baseline level indicative of NASH of at least one criteria selected from the group consisting of NAS score, steatosis score, lobular inflammation score, ballooning score and fibrosis stage. In particular, the patient has baseline levels of ALT of between 5 to 300 and at least one criteria selected from the group consisting of NAS score of ≥ 4, a steatosis score of ≥ 1, a lobular inflammation score of ≥ 1 and either (i) a fibrosis stage of at least 1a or (ii) ballooning. Whether a patient is in need of treatment of NASH may also be determined prior to treatment by measuring the baseline level in blood or the physical condition of at least one member selected from the group consisting of ALT, AST, AST/ALT ratio, ALP, bilirubin, GGT, Albumin, HDL-C, LDL-C, TG, TC, TG/HDL-C ratio, non-HDL-C, Free fatty acid, AA, EPA, DPA, DHA, EPA/AA ratio, DPA/AA ratio, DHA/AA ratio, DHA/DPA ratio, MUFA, Palmitoleic acid, Oleic acid, Oleic acid/Stearic acid ratio, Palmitoleic acid/Palmitic acid ratio, Stearic acid/Palmitic acid ratio, γ-linoleic acid/Linoleic acid ratio, AA/Homo-γ-linoleic acid ratio, Adrenic acid/AA ratio, Ferritin, Thioredoxin, TNF α, sTNF-R1, sTNF-R2, Hs-CRP, CTGF, sCD40, HOMA-IR, HbA1c, Glucose, Fasting plasma glucose, postprandial plasma glucose, OGTT, Leptin, Serum adiponectin, complement factor D, CK18 fragment, serum HMGB1, soluble Fas antigen, Hyaluronic acid, Type IV collagen (7s domain), procollagen III peptide, PAI-1, platelet count or BMI, or at least one criteria (marker) of the items mentioned in Tables 1 and 2.

After administration of the compound or pharmaceutical composition for use according to the present invention, the subject (a patient in need of treatment of NASH) may show an improvement in the NAS score (i) to a composite score of less than or equal to 3 or (ii) by 2 across at least two of the NAS components, combined with no worsening of the fibrosis stage score.

Alternatively, the subject may show an improvement in the NAS score (i) to a composite score of ≤ 3 or (ii) by ≥ 2 across at least two of the NAS components, together with no worsening of the fibrosis stage score.

Further, the subject may show an improvement in at least one condition selected from the group consisting of the steatosis, lobular inflammation, ballooning and liver fibrosis condition of said subject, and no worsening of fibrosis stage score; and said subject may exhibit the following changes in at least one of the following markers as compared to a baseline pre-treatment level: at least 1% reduction for ALT, AST, Triglycerides (TG), TG/HDL-C ratio, Free fatty acid, Arachidonic acid (AA), monounsaturated fatty acid (MUFA), Palmitoleic acid, Oleic acid, Oleic Acid/Stearic acid ratio, Palmitoleic acid/Palmitic acid ratio, Stearic acid/Palmitic acid ratio, γ-linoleic acid/Linoleic acid ratio, Adrenic acid/AA ratio, Ferritin, Thioredoxin, TNF α, sTNF-R1, sTNF-R2, Hs-CRP, CTGF, sCD40, Leptin, complement factor D, CK18 fragment, serum HMGB1, soluble Fas antigen, Hyaluronic acid, Type IV collagen (7s domain), procollagen III peptide or PAI-1; at least 5% increase for EPA or EPA/AA ratio; at least 1% increase for DPA, AA/Homo-γ-linoleic acid ratio or Serum adiponectin; no worsening of ALP, bilirubin, GGT, Albumin, HDL-C, LDL-C, Total Cholesterol (TC), non-HDL-C, HOMA-IR, HbA1c, Fasting plasma glucose, postprandial plasma glucose, OGTT, platelet count or BMI.

Alternatively, the subject may exhibit the following changes in at least one marker as compared to its baseline pre-treatment level: at least 1% reduction for ALT, AST, TG, TG/HDL-C ratio, Free Fatty acid, AA, MUFA, Palmitoleic acid, Oleic acid, Oleic acid/Stearic acid ratio, Palmitoleic acid/Palmitic acid ratio, Stearic acid/Palmitic acid ratio, γ-linoleic acid/Linoleic acid ratio, Adrenic acid/AA ratio, Ferritin, Thioredoxin, TNF α, sTNF-R1, sTNF-R2, Hs-CRP, CTGF, sCD40, Leptin, complement factor D, CK18 fragment, serum HMGB1, soluble Fas antigen, Hyaluronic acid, Type IV collagen (7s domain), procollagen III peptide or PAI-1; at least 5% increase for EPA or EPA/AA ratio; at least 1% increase for DPA, AA/Homo-γ-linoleic acid ratio or Serum adiponectin; no worsening of ALP, bilirubin, GGT, Albumin, HDL-C, LDL-C, TC, non-HDL-C, HOMA-IR, HbA1c, Glucose, Fasting plasma glucose, postprandial plasma glucose, OGTT, platelet count or BMI; or change in at least one criteria (marker) of the items mentioned in Table 2 as defined therein.

Further, the subject may show a reduction of at least one marker as compared to a baseline pre-treatment level of Hs-CRP, CTGF, sCD40, Leptin, complement factor D, serum HMGB1, soluble Fas antigen or procollagen III peptide.

Furthermore, the subject may show an improvement or no worsening in at least one of the markers given Table 1.

The patient in need of treatment of NASH may be taking anti-diabetic drugs. He/she may be diabetic or not diabetic.

Pharmaceutical compositions comprising EPA-E for use according to the invention include commercially available compositions of EPA-E, such as Epadel® noted above. The compositions comprising EPA-E may be administered in tablet, capsule, microcapsule, jelly, enteric preparation, extended release preparation, powder or any other solid oral dosage form, as a liquid, emulsion, self-emulsifying composition, as a soft gel capsule or other capsule form, or other appropriate and convenient dosage forms for administration to a patient in need thereof. The compositions can also include pharmaceutically acceptable excipients including surfactants, oils, cosolvents or combinations of such excipients, together with stabilizers, emulsifiers, preservatives, solubilizers and/or other non-active pharmaceutical ingredients.

An exemplary soft capsule containing EPA-E is a commercially available soft capsule containing high purity EPA-E which is sold in Japan in the product name of Epadel and Epadel S (both manufactured by Mochida Pharmaceutical Co., Ltd.) as a safe therapeutic agent for arteriosclerosis obliterans and hyperlipidemia exhibiting reduced side effect expression, and the content ratio of the EPA-E to the entire fatty acid is at least 96.5% by weight. Another exemplary soft capsule containing about 46% by weight of EPA-E and about 38% by weight of DHA-E (Omacor™ by Ross Products) is sold in, for example, U.S. as a therapeutic agent for hypertriglycemia. These may be obtained and used.

The pharmaceutical compositions of the present invention may further contain optional components such as excipient, binder, lubricant, colorant, flavor, sterilized water, vegetable oil, harmless organic solvent, harmless solubilizing agent (such as glycerin and propylene glycol), emulsifier, suspending agent (for example, Tween 80 and gum arabic solution), isotonic agent, pH adjusting agent, stabilizer, and soothing agent.

Since EPAs are highly unsaturated, the pharmaceutical composition for use according to the present invention preferably contains an antioxidant at an amount effective for suppressing oxidation of the EPA-E. Exemplary antioxidants include butylated hydroxytoluene (BHT), butylated hydroxyanisole (BHA), propyl gallate, gallic acid, pharmaceutically acceptable quinone, and α-tocopherol.

### 1. Patients in need of treatment of NASH

As noted above, the "gold-standard" for a complete diagnosis of NASH involves a liver biopsy. The patients or subjects in need of treatment for NASH can also be evaluated for the following criteria, including evaluation prior to initiation of treatment in order to provide a baseline level or score for the criteria as well as evaluation after the dosing regimen to evaluate any improvement in the criteria.

### a. NAS Score

A non-alcoholic fatty liver disease activity score (NAS) is defined as the unweighted sum of the values for steatosis (ranging from 0-3), lobular inflammation (ranging from 0-3) and ballooning (ranging from 0-2), thereby providing a range of NAS score of from 0 to 8. (See Kleinen et al., Design and Validation of a Histological Scoring System for Nonalcoholic Fatty Liver Disease, Hepatology, Vol. 41, No. 6, 2005, pp. 1313-1321) Patients in need of treatment for NASH can show a NAS score prior to treatment of ≥ 4, with a minimum score of 1 each for steatosis and lobular inflammation plus either ballooning or at least 1a sinusoidal fibrosis and a finding of possible or definite steatohepatitis. After dosing/treatment, such as for one year, patients can show a composite NAS score of ≤ 3, ≤ 2 or ≤ 1, together with no worsening in fibrosis. Alternatively, patients can show an improvement in NAS by a value of ≥ 2 across at least two of the NAS components, together with no worsening in fibrosis. Alternatively, patients can show an improvement in NAS score by ≥ 3, 4, 5, 6, 7 or 8.

### b. Steatosis

Steatosis is broadly understood to describe a process involving the abnormal retention of lipids within the liver, whose accumulation inhibits the normal liver functions. Liver biopsy enables analysis and scoring of steatosis in a patient, with scores ranging from 0-3. The patients in need of treatment for NASH can have a steatosis score of 1, 2 or 3, such as between about 2 and about 3. After treatment, it is desired for patients to exhibit no worsening of steatosis, alternatively a reduction of at least 1 in the steatosis score, or a reduction of 2 or 3 in the steatosis score. Steatosis is traditionally graded with a score of 1 indicating the presence of fat droplets in less than 33% of hepatocytes, a score of 2 indicating fat droplets observed in 33-66% of hepatocytes, and a score of 3 indicating observation of fat droplets in greater than 66% of hepato sites. (See Kleinen et al., Design and Validation of a Histological Scoring System for Nonalcoholic Fatty Liver Disease, Hepatology, Vol. 41, No. 6, 2005, pp. 1313-1321)

### c. Lobular Inflammation

Lobular inflammation is also evaluated upon liver biopsy and scored with values of 0-3. (See Kleinen et al., Design and Validation of a Histological Scoring System for Nonalcoholic Fatty Liver Disease, Hepatology, Vol. 41, No. 6, 2005, pp. 1313-1321 Table 1) The patients in need of treatment for NASH can have lobular inflammation scores of 1, 2 or 3, alternatively ranging between 1 and 2 or 2 and 3. After treatment, patients can have a reduction in lobular inflammation score of at least 1, alternatively a reduction of 2 or 3 in lobular inflammation score, and at least no worsening of the lobular inflammation score.

### d. Ballooning

Ballooning of hepatocytes is generally scored with values of 0-2, (See Kleinen et al., Design and Validation of a Histological Scoring System for Nonalcoholic Fatty Liver Disease, Hepatology, Vol. 41, No. 6, 2005, pp. 1313-1321 Table 1), and patients in need of treatment for NASH can have ballooning scores of 0-2, including specific values of 1 or 2, and alternatively a score ranging from 1 to 2. After treatment, patients can show at least no worsening of the ballooning score, alternatively a reduction of at least one value lower in the ballooning score, and alternatively a reduction of two in the value of the ballooning score.

### e. Fibrosis Stage

Fibrosis is also evaluated upon liver biopsy and scored with values of 0-4, the scores being defined as: 0 represents no fibrosis, 1 represents perisinusoidal or periportal fibrosis, 1a represents mild, zone 3, perisinusoidal fibrosis; 1b represents moderate zone 3, perisinusoidal fibrosis; 1c represents portal/periortal fibrosis; 2 represents perisinusoidal and portal/periportal fibrosis; 3 represents bridging fibrosis; and 4 represents cirrhosis. (See Kleinen et al., Design and Validation of a Histological Scoring System for Nonalcoholic Fatty Liver Disease, Hepatology, Vol. 41, No. 6, 2005, pp. 1313-1321) The patients in need of treatment for NASH can have a fibrosis stage score of 0-3, including 0, 1, 1a, 1b, 1c, 2 or 3, and can have a fibrosis stage score of at least 1 a. After treatment, patients can have a fibrosis stage score that is at least no worse than the baseline score, and alternatively can have a reduction in the fibrosis stage score of at least one level, alternatively at least two or three levels.

### 2. Additional Criteria/Markers for Evaluation of Patients

As noted above, while liver biopsy is considered the "gold-standard" for clinical assessment of NASH, the condition can also be accompanied or associated with abnormal levels of liver enzymes and other biological blood components. Therefore, patients in need of treatment for NASH can also be evaluated for baseline scores of the following criteria before treatment, and evaluated after treatment for possible changes in those criteria. The evaluated criteria can comprise one or more of the following criteria set forth in Tables 1 and 2.

**Table 1**

| **Item (Typical Normal Values, Units)** | **Pre-treatment baseline** | | **After dosing (effect) values** | |
|---|---|---|---|---|
| | **Typical Range(s)** | **Observable Ranges or Values** | **Typical Range(s)** | **Observable Ranges or Values** |
| ALT (alanine aminotransfer ase, GPT) (6-41 U/L) | 10-300 | Lower limit range values of 10, 50, 100, 150, or 200, upper limit range values of 100, 150, 200, 250, or 300, ranges of 10-300, 10-200, 10-150, 10-100, 100-200, 2000-3000 | at least 1% lower | 1 to about 95% reduction |
| AST (asparate aminotransfer ase,GOT) (9-34 U/L) | 10-250 | Lower limit range values of 10, 50, 100, 150, or 200, upper limit range values of 100, 150, 200, 250, or 300, ranges of 10-300, 10-200, 10-150, 10-100, 100-200, 200-300 | at least 1% lower | 1 to about 95% reduction |
| AST/ALT ratio | | upper limit range values of 0.5, 0.7, 0.8, 1, 1.2, 2; ranges of 0.5-2, 0.5-1-1-2 | | |
| alkaline phospatase (ALP) (80-260 IU/L) | 80-300 | ranges of 50-600 | no worsening | no worsening, 1 to about 90% reduction, 300IU/L or less, 250IU/L or less |
| Total bilirubin (0.2-1.2 mg/dL) | High compared to average level of normal subject | | no worsening | no worsening, 1 to about 90% reduction |
| Gamma-Glutamyl Transferase (GGT or γGTP) (males: 5-60 U/L) | High compared to average level of normal subject | | no worsening | no worsening, 1 to about 90% reduction, 100U/L or less, 70U/L or less |
| Albumin (3.8-5.2 g/dl) | Low compared to average level of normal subjects | | no worsening | no worsening, 1 to about 90% increase, ranges of 3-6 g/dl, 3.5-5.5 g/dl |
| HDL-C (high density lipoprotein cholesterol) (35-60- mg/dl) | less than 55 | less than 60mg/dl, 55, 50, 45, 40, 35, 30, 25, or 25 mg/dl; ranges of 25-55, 30-40mg/dl, 40-50mg/dl, 50-60mg/dl, at least 60 | no worsening, at least 1% increase | no change, 1-90% increase, 40 mg/dl or more |
| LDL -C (low density lipoprotein cholesterol) (50-130 mg/dl) | 100-200 | at least 70 mg/dl, 100 , 120, 130 140 150, 170, 190, or 200 or a range of 70-300, 70-250, 70-200, 100-250, 100-200, 130-200, 140-180, 100-130, 130-160, 160-190 | no worsening | no change, 1-90% reduction less than 160mg/dl, 140, 130, 120, 100, 70mg/dl |
| Triglycerides (TG) (fed or fasting, 50-150mg/dl) | 100-1000 | at least 80 mg/dl, 100, 150, 180, 200, 300, 500, 700, 1000, 1200, or 1500, or less than 150, or a range of 100-2500, 100-1500, 100-1000, 150-500, 200-500, 150-300, 150-200, 200-500 | at least 1% lower | 1 to about 90% reduction, 500 mg/dl or less, 300, 200, 150, 100mg/dl or less |
| Total Cholesterol (TC) (100-200 mg/dl) | 170-300 | a range of 130-300 mg/dl, 200-220, 220-240, 240-260, or at least 260, or less than 200 mg/dl | no worsening | no change, 1-90% reduction |
| TG and HDL-C | High TG and low HDL-C (ex. TG ≥ 150mg/dl and HDL≤40mg/dl | TG: at least 150, 200, 500mg/dl HDL-C; less than 40, 50 mg/dl | no worsening | |
| TG/HDL-C ratio | at least 3.75 | at least 2, 2.5, 3, 3.75, 4, 5, 10, or ranges of 2-3.75, 3.75-10 | at least 1% lower | no worsening, at least 1% lower, or 1-90% reduction |
| Non-HDL-C (mg/dl) | at least 130 | at least 100mg/dl, 130, 150, 160, 170, 190, a range of 100 to 250 | no worsening | no worsening, or at least 1% lower, or less than 130mg/dl, 150, 160, 170, 190 |
| Free fatty acid (µ Eq/l) (140-850) | at least 400 | less than 400, at least 400, 600, 800, 1000 | at least 1% lower | no change, or at least 1 to 90% reduction |
| Eicosapentaen oic Acid/Arachido nic Acid (EPA/AA) (ex. (mol/%)/ (mol/%) | less than 0.5 / low compared to average level or normal subjects | less than 1, 0.75, 0.5, 0.1, ranges of 0.01-2 | at least 5% increase | 5 to about 200% increase, about 2-200-fold increase |
| Arachidonic Acid (AA) (ex. mol/%) | High compared to average level of normal subjects | | at least 1% lower | no change, 1 to about 90% reduction |
| Eicosapentaen oic Acid (EPA) (ex. mol/%) | low compared to average level of normal subjects | | at least 5% increase | 5 to about 200% increase, about 2-500-fold increase |
| Docosapentae noic Acid (DPA) (ex. mol/%) | low compared to average level of normal subjects | | at least 1% increase | 1 to about 95% increase |
| Docosahexaen oic Acid (DHA) (ex. mol/%) | low compared to average level of normal subjects | | | |
| DPA/AA ratio | low compared to average level of normal subjects | | | |
| DPA/AA ratio | low compared to average level of normal subjects | | | |
| DHA/DPA ratio | low compared to average level of normal subjects | | | |
| Monounsatur ated fatty acid (MUFA) (ex. mol/%) | High compared to average level of normal subjects | | at least 1% lower | no change, at least 1% lower |
| Palmitoleic acid (16:1 n7) (ex. mol/%) | High compared to average level of normal subjects | | at least 1% lower | no change, at least 1% lower |
| Oleic acid (18:1 n9) (ex. mol/%) | High compared to average level of normal subjects | | at least 1% lower | no change, a least 1% lower |
| Oleic acid (18:1 n9) /stearic acid (18:0) ratio | High compared to average level of normal subjects | | at least 1% lower | no change, at least 1% lower |
| Palmitoleic acid (16:1) /Palmitic acid (16:0) ratio | High compared to average level of normal subjects | | at least 1% lower | no change, at least 1% lower |
| Stearic acid (18:0)/ Palmitic acid (16:0) ratio | High compared to average level or normal subjects | | no change, or at least 1% lower | no change, or at least 1% lower |
| γ-linoleic acid(18:3 n6) /Linoleic acid (18:2 n6) ratio | High compared to average level subjects | | no change, or at least 1% lower | no change, or at least 1% lower |
| AA/Homo-γ-linolenic acid (20:3 n6) ratio | low compared to average level of normal subjects | | no change, or at least 1% increase | no change, or at least 1% increase |
| Acrenic acid (22:4 n6) /AA ratio | High compared to average level of normal subjects | | no change, or at least 1% lower | no change, or at least 1% lower |
| Ferritin (ng/mL) | | at least 100, 120, 150, 200, 250, 300, 350, 400, or 500 | at least 1% lower | at least 1 to about 95% lower |
| Thioredoxin (ng/mL) | | at least 15, 20, 25, 30, 35, 40, 45, or 50 | at least 1% lower | at least 1 to about 95% lower |
| TNFα (Tumor necrosis factor-α) (pg/mL) (1.79 or less) | at least 1.5 | at least 1, 1.5, 1.6, 1.7, 1.79, 1.8, 1.9, 2.0, 2.2, 2.5, 3, 3.5, 4, 5, 6, 7 or 10 | at least 1% lower | at least 1 to about 95% lower |
| sTNF-R1 (Tumor necrosis factor receptor I , s oluble) (pg/mL) | | at least 400, 500, 600, 700, 800, 900, 1000, 1100, 1200, 1500, or 2000 | at least 1% lower | at least 1 to about 95% lower |
| sTNF-R2 (Tumor necrosis factor receptor II, soluble) (pg/mL) | | at least 500, 700, 1000, 1200, 1500,1700, 2000, 2200, 2500, 2700, or 3000 | at least 1% lower | at least 1 to about 95% lower |
| High Sensitivity C-reactive protein (Hs-CRP, mg/dl) | 0.2 | 0.1 or more, 0.2, 0.3, 0.4, 0.5 or more, ranges of 0.1-1, 0.1-0.8, 0.1-0.5, 0.2-0.5 | at least 1% lower | at least 5 to about 95% lower |
| Connective Tissue Growth Factor (CTGF) | High compared to average level of normal subject | | at least 1% lower | at least 5 to about 95% lower |
| Serum Soluble CD40 (sCD40, pg/ml) | | 5 pg/ml or more, 10, 20, 30, 50, 70, 100, 120, 150, 170, 200, 220, 250, 300, 350, 400, 450, 500 or more | at least 1% lower | at least 5 to about 95% lower |
| Insulin resistance Index (HOMA-IR) (1.6 or less) | 1.5 or more | 1.6 or less/ 1.5 or more, 1.6, 2, 2.5, 3, 3.5, 4 | no worsening | no change, at least 1 to about 50% lower |
| Glycated hemoglobin (HbA1c) (4.3-5.8%) | 5.7 or more | a range of 4.3-5.8, 5.7-6.4, 5.8-6.5, 6.5-7.0, 7.0-8.0/ 5.7 or more, 5.8, 6, 6.5, 7, 7.5, 8, or 8.5 | no worsening | no change, at least 1 to about 50% lower |
| Fasting plasma glucose (FPG) (mg/dl) (less than 100) | 100 or more | less than 100 / 100 or more, 110, 120, 126, 130, 150, 200, 250, 300 / ranges of 100-110, 100-126 | no worsening | no change, or at least 1 to about 50% lower |
| Postprandial plasma glucose (after a meal) | 140 or more | less than 140, 160, 200/ 140 or more, 170, 180, 200, 250,300,350 400 / ranges of 140-200,140-170, 170-200 | no worsening | no change, or at least 1 to about 50% lower |
| two-hour glucose levels on the 75-g oral glucose tolerance test (mg/dl) (OGTT) | 140-200 | less than 140, 160, 200/ 140 or more, 170, 180, 200, 250, 300, 350, 400/ ranges of 140-200,140-170, 170-200 | no worsening | no change, or at least 1 to about 50% lower |
| Leptin (ng/ml) | | 5 ng/ml or more, 10, 12, 15, 7, 20, 22 ,25, 30, 35, 40 or more | at least 1% lower | at least 1 to about 95% reduction |
| Serum adiponectin (µg/mL) | | 5 µg/mL or less, 4.5, 4, 3.5, or 3 µg/mL or less | at least 1% increase | no change, at least 1 to about 95% increase |
| complement factor D | High compared to average level of normal subject | | at least 15% lower | at least 1 to about 95% reduction |
| CK18 fragment | High compared to average level of normal subject | | at least 1% lower | at least 1 to about 95% reduction |
| serum High mobility group box 1 protein (HMGB1) | High compared to average level of normal subject | | at least 1% lower | at least 1 to about 95% reduction |
| soluble Fas antigen (CD95, sFas) | High compared to average level of normal subject | | at least 1% lower | at least 1 to about 95% reduction |
| Hyaluronic acid (50ng/mL or less) | | 25ng/mL or more, 50, 70, 100, 120, 150, 200, 250, or 300 or more; 200mL or less, 100, 70, or 50 or less | at least 1% lower | at least 1 to about 95% reduction |
| Type IV collagen (7s domain) (6ng/mL or less) | | 5ng/mL or more, 6, 7, 8, 10, 12, 15, or 20 or more; 25ng/mL or less, 20, 15, 10, or 6 or less | at least 1% lower | at least 1 to about 95% reduction |
| procollagen III peptide 0.3-0.8U/ml | | 0.2 U/ml or more, 0.3, 0.5, 0.7, 1, 1.2, 1.5, 2, 2.5, 3, 3.5, or 4 or more; 10 or less, 8, 5, 3, 1, or 0.8 or less | at least 1% lower | at least 1 to about 95% reduction |
| PAI-1 (ng/mL) 50 or less | 50 or more | | | |
| platelet count 150000-400000/µL | 150000-300000 | 400000/µL or less, 300000, 200000/ a range of 150000-300000 | no change | no change, at least 1% increase |
| BMI | 18.5-40 | 18.5 or more, 20, 25, 30, 35, 40, or 50 or more;/ 50 or less, 40, 30, 25, 20 or 18.5 or less; or range of 18.5-25, 25-30, 30-35. 35-40 | no change | no change, at least 1% reduction |
| Direct Bilirubin (0-0.4mg/dl) | High compared to average level of normal subject | | No worsening | No worsening, 1 to about 90% reduction |
| Oleic acid (C18:1 n9)/ Palmitic acid (C16:0)ratio | High compared to average level of normal subject | | At least 1% lower | No change, at least 1% lower |
| EPA/AA ratio and Hs-CRP | Low EPA/AA ratio and high Hs-CRP | EPA/AA ratio being 1.0 or less, 0.75, 0.6, 0.5, 0.4, 0.25 or less; Hs-CRP being 0.1mg/dl or higher, 0.2mg/dl or higher, 0.3mg/dl or higher | | EPA/AA ratio increases; Hs-CRP decreases |
| Interleukin-1 receptor antagonist (IL-1 ra) | High compared to average level of normal subject | | at least 1% lower | at least 1 to about 95% lower |
| sPLA2(Secretory phospholipase A2) group II A : type2A, type II A | High compared to average level of normal subject | | at least 1% lower | at least 1 to about 95% lower |
| sPLA2 activity | Low compared to average level of normal subjects | | No worsening | |
| Interleukin 2(IL-2) | High compared to average level of normal subject | | at least 1% lower | at least 1 to about 95% lower |
| ApolipoproteinA-IV | High compared to average level of normal subject | | at least 1% lower | at least 1 to about 95% lower |
| ApolipoproteinC-II | High compared to average level of normal subject | | at least 1% lower | at least 1 to about 95% lower |
| CCL2: Chemokine(C-C motif) ligand 2 | High compared to average level of normal subject | | at least 1% lower | at least 1 to about 95% lower |
| Thrombospondin 1 :TSP1 | High compared to average level of normal subject | | at least 1% lower | at least 1 to about 95% lower |
| IL-3 receptor (Interleukin-3 receptor) alpha chain | High compared to average level of normal subject | | at least 1% lower | at least 1 to about 95% lower |
| Lymphocyte antigen 6 comlex, locus D | High compared to average level of normal subject | | at least 1% lower | at least 1 to about 95% lower |
| MMP12: Matrix metallopeptidas e 12 | High compared to average level of normal subject | | at least 1% lower | at least 1 to about 95% lower |
| MMP13: Matrix metallopeptidas e13 | High compared to average level of normal subject | | at least 1% lower | at least 1 to about 95% lower |
| Trehalase (brush-border membrane glycoprotein) | High compared to average level of normal subject | | at least 1% lower | at least 1 to about 95% lower |
| TIMP1: Tissue inhibitor of metalloproteinas e 1 | High compared to average level of normal subject | | at least 1% lower | at least 1 to about 95% lower |
| COL1a1: Procollagen type I , alpha 1 | High compared to average level of normal subject | | at least 1% lower | at least 1 to about 95% lower |
| Complement factor D (adipsin) | High compared to average level of normal subject | | at least 1% lower | at least 1 to about 95% lower |
| TNFR (tumor necrosis factor receptor) superfamily, member 19 (TAJ) | High compared to average level of normal subject | | at least 1% lower | at least 1 to about 95% lower |
| TNFAIP (tumor necrosis factor alpha induced protein) 6 | High compared to average level of normal subject | | at least 1% lower | at least 1 to about 95% lower |
| VLDLR (Very low density lipoprotein receptor) | High compared to average level of normal subject | | at least 1% lower | at least 1 to about 95% lower |
| Lipoprotein lipase | High compared to average level of normal subject | | at least 1% lower | at least 1 to about 95% lower |
| Ear (Eosinophil associated ribonuclease) A family, members 1,2,3, and 12 | High compared to average level of normal subject | | at least 1% lower | at least 1 to about 95% lower |
| INSL5: Insulin like 5 | Low compared to average level of normal subjects | | At least 1% increase | |
| TGFβ 2 : Transforming growth factor beta 2 | Low compared to average level of normal subjects | | At least 1% increase | |
| HAMP: Hepcidin antimicrobial peptide 1 | Low compared to average level of normal subjects | | At least 1% increase | |
| Lipase member H: LIPH | Low compared to average level of normal subjects | | At least 1% increase | |
| CYP7B1: Cytochrome P450 family 7 subfamily b polypeptide 1 | Low compared to average level of normal subjects | | At least 1% increase | |

**Table 2**

| Item (Typical Normal Values, Units) | Pre-treatment baseline | | After dosing (effect) values | |
|---|---|---|---|---|
| | Typical Range(s) | Observable Ranges or Values | Typical Range(s) | Observable Ranges or Values |
| 1 1 - HETE (11-hydroxy-5,8,12,14-eicosatetraenoi c acid) | High compared to average level of normal subject | | at least 1% lower | at least 1 to about 95% lower |
| Total HEPEs (hydroxyeicosap entaenoic acids) /total HETEs (Hydroxyeicosat etraenoic Acids) ratio | Low compared to average level of normal subjects | | At least 1% increase | |
| Glycocholate | High compared to average level of normal subject | Twice or more than twice as high as normal subject | at least 1% lower | |
| Taurocholate | High compared to average level of normal subject | Twice or more than twice as high as normal subject | at least 1% lower | |
| Glycocholate/GI ycine ratio | High compared to average level of normal subject | Twice or more than twice as high as normal subject | at least 1% lower | |
| Taurocholate/Ta urine ratio | High compared to average level of normal subject | Twice or more than twice as high as normal subject | at least 1% lower | |
| Total fatty acids of 20 to 24 carbon atoms (C20-24)/total fatty acids of 16 carbon atoms (C16) ratio (ex. /µg/mlµg/ml, wt%/wt%) | Low compared to average level of normal subjects | | At least 1% increase | |
| Total omega-3 polyunsaturated fatty acids of 20 to 24 carbon atoms(C20-24) /total fatty acids of 16 carbon atoms (C16) ratio (ex. µg/ml /µg/ml, wt%/ wt%) | Low compared to average level of normal subjects | | At least 1% increase | |
| Total fatty acids of 20 to 24 carbon atoms(C20-24) /total fatty acids of 18 carbon atoms (C18) ratio (ex. µg/ml /µg/ml, wt%/ wt%) | Low compared to average level of normal subjects | | At least 1% increase | |
| Total omega-3 polyunsaturated fatty acids of 20 to 24 carbon atoms(C20-24) / total weight of fatty acids of 18 carbon atoms (C18) ratio (ex. µg/ml/µg/ml, wt%/wt%) | Low compared to average level of normal subjects | | At least 1% increase | |
| IL-10(Interleukin-10) | No change or Low compared to average level of normal subjects | | At least 1% increase | |
| Small dense LDL | No change or High compared to average level of normal subjects | at least 20mg/dl, 25, 30, 40, 50, at least 60mg/dl | at least 1% lower | |
| RLP-TG (Remnant-like lipoprotein particles-triglyceride) | No change or High compared to average level of normal subjects | at least 10 mg/dl, 20, 30, 40, 50, 70, 80, 100, 120, at least 150 mg/dl | at least 1% lower | |
| RLP-C (Remnant-like lipoprotein particles-cholesterol) | No change or High compared to average level of normal subjects | At least 4.5mg/dl, 5, 5.2, 5.5, 6, 8, 10, 12, at least 15 mg/dl | at least 1% lower, or no change | |
| Whole Blood viscosity (cP/mPa · s) | | High compared to average level of normal subject | at least 1% lower | at least 1% lower |
| Plasma viscosity (cP/mPa · s) | | High compared to average level of normal subject | No worsening | |
| IL-10 (Interleukin-10) /TNFα ratio | Low compared to average level of normal subject | | At least 1% increase | |
| IL-10 (Interleukin-10) /sCD40 ratio | Low compared to average level of normal subject | | At least 1% increase | |
| Serum /adiponectin TNFα ratio | Low compared to average level of normal subject | | At least 1% increase | |
| Serum adiponectin/ sCD40 ratio | Low compared to average level of normal subject | | At least 1% increase | |

Unless otherwise specified, the fatty acid amount and the fatty acid composition ratio as used in the present invention may be the amount and the composition ratio of fatty acids in any of the plasma, serum and liver. It is also possible indeed to use the fatty acid amount and the fatty acid composition ratio in a specified fraction, such as LDL or VLDL in the blood. It, however, is desirable to use the amount and the composition ratio of fatty acids in the plasma or the serum because of the simplicity of measurement. Each fatty acid to be employed for the calculation of the fatty acid amount and the fatty acid composition ratio is not particularly limited in unit of amount, that is to say, its amount may be expressed in mole, mole percent, a unit of weight, percent by weight, or the like. The sole unit, and the sole method of calculating fatty acid amount and the fatty acid composition ratios should be used if the evaluation is to be made by the comparison of the fatty acid amount and the fatty acid composition ratio over time. It is particularly desirable to calculate the fatty acid amount and the fatty acid composition ratio from fatty acid amounts expressed in mole percent of the total amount of fatty acids. The weight/volume concentration (e.g., micro g/ml), the mole/volume concentration (e.g., mol/L) or the like may also be used for the calculation.

In this description, the term "plasma fatty acid" refers to a plasma total fatty acid unless otherwise specified. It is also possible to use a plasma free fatty acid for the inventive index for the evaluation of the subject's condition or therapeutic effects. The term "liver fatty acid" refers to a liver total fatty acid unless otherwise specified. A liver free fatty acid may optionally be used.

The fatty acid composition may be determined by any method practicable by a person of ordinary skill in the art of the present invention, while it is particularly preferable to determine the composition according to a usual manner.

Measurement values in the present invention are taken from a biological sample of a subject. Specific examples of the biological sample collected from a subject include blood, plasma, serum, urine, body fluids, and tissues, but are not limited thereto. The biological sample is preferably blood, plasma or serum. The biological sample is collected from a subject by a known method.

In the present invention, a normal value is measured in accordance with a known measuring method if the normal value is known as one of the blood test indices used to detect NASH, or in accordance with a measuring method following a reference document or the like if a common measuring method for the normal value is not established. Reference documents describing measuring methods used in the present invention are given in Table 3.

**Table 3**

| | |
|---|---|
| 11-HETE | Prostaglandins Other Lipid Mediat. 2011 Apr;94(3-4):81-7. |
| HETE, HEPE | Analysis of omega-3 and omega-6 fatty acid-derived lipid metabolite formation in human and mouse blood samples. |
| Glycocholate | Metabolism. 2011 Mar;60(3):404-13. |
| Taurocholate | Plasma metabolomic profile in nonalcoholic fatty liver disease. |
| IL-10 | Obes Surg. 2010 Jul;20(7):906-12. |
| | Pro- and anti-inflammatory cytokines in steatosis and steatohepatitis. |
| Small dense LDL | Diabetol Metab Syndr. 2012 Jul 18;4(1):34. |
| | Fatty liver in men is associated with high serum levels of small, dense low-density lipoprotein cholesterol. |
| RLP-TG | Clinica Chimica Acta 413 (2012) 1077-1086 |
| RLP-C | The characteristics of remnant lipoproteins in the fasting and postprandial plasma. |
| Connective Tissue Growth Factor (GTGF) | Regul Pept. 2012 Sep 4;179(1-3):10-14. |
| | Connective tissue growth factor level is increased in patients with liver cirrhosis but is not associated with complications or extent of liver injury. |
| Serum Soluble CD40 (sCD40) | Apoptosis 2004;9: 205-210 |
| | Role of circulating soluble CD40 as an apoptotic marker in liver disease. |
| Complement factor D | Int Immunopharmacol. 2009 Nov;9(12): 1460-3. |
| | Serum adipsin levels in patients with seasonal allergic rhinitis: preliminary data. |
| CK18 fragment | Aliment Pharmacol Ther. 2010 Dec;32(11-12):1315-22. |
| | A new composite model including metabolic syndrome, alanine aminotransferase and cytokeratin-18 for the diagnosis of non-alcoholic steatohepatitis in morbidly obese patients. |
| Serum High mobility group box 1 protein (HMGB1) | PLoS One. 2012;7(4):e34318. |
| | Diagnostic significance of serum HMGB1 in colorectal carcinomas. |
| fatty acid amount and fatty acid composition ratio in blood | Clinical Nutrition (2002) 21 (3) 219-223 |
| | Plasma total and free fatty acids composition in human non-alcoholic steatohepatitis. |
| Ferritin, Thioredoxin | J Hepatol. 2003 Jan;38(1):32-8. |
| | Serum thioredoxin levels as a predictor of steatohepatitis in patients with nonalcoholic fatty liver disease. |
| sTNF-R1, sTNF-R2 | Diabetes Care. 2010 Oct;33(10);2244-9. Epub 2010 Jul 27. |
| | Association between systemic inflammation and incident diabetes in HIV-infected patients after initiation of antiretroviral therapy. |
| Hs-CRP | J Hepatol. 2011 Sep;55(3):660-5. |
| | C-reactive protein levels in relation to various features of non-alcoholic fatty liver disease among obese patients. |
| soluble Fas antigen (CD95, sFas) | J Transl Med. 2009 Jul 29;7:67. |
| | Short term effects of milrinone on biomarkers of necrosis, apoptosis, and inflammation in patients with severe heart failure. |
| Whole Blood viscosity Plasma viscosity | British Journal of Haematology,1997 96, 168-173 Blood viscosity and risk of cardiovascular events: the Edinburgh Artery Study |
| Items of Table1(1-8, 1-9, 1-10) | W02011 / 046204 |

### Example (Double-blind, placebo controlled study of the effect of two different doses of EPA-E on NASH)

Patients were evaluated for inclusion in the treatment regimen, treated for NASH, and evaluated for effectiveness of the treatment as follows:

Patients are histologically diagnosed with NASH within six months of the initiation of treatment and are willing to submit to a further liver biopsy at the end of the treatment regimen to evaluate effectiveness of the treatment.

### 1. Inclusion Criteria:

Patients are definitively diagnosed with NASH (via liver biopsy) and exhibit a NAS score of greater than or equal to 4 by a pathologist.
● Patients can be of either gender but are greater than 18 years of age.
● Patients with diabetes, impaired glucose tolerance or metabolic syndrome who have been on stable dosage of anti-diabetic agents for at least six months prior to the liver biopsy are suitable for the study.

### 2. Exclusion Criteria:

Patients are excluded from participation in the study based upon an inability or unwillingness to have a liver biopsy for confirming the diagnosis of NASH, having a diagnosis of cirrhosis by a pathologist, exhibiting previous bariatric surgery or biliary diversion (*i*.*e*. gastric bypass), esophageal banding or gastric banding; serum ALT values of greater than 330 UL, drug use associated with steatohepatitis within 6 months prior to initiation of treatment, such as with corticosteroids, high dose estrogens, methodtrexate, amiodarone, anti-HIV drugs, tamoxifen, or diltiazem; alcohol consumption of greater than 30 g/day, concurrently or for more than three consecutive months within five years prior treatment; a blood alcohol level greater than 0.02% at the time of baseline evaluation; evidence of active substance abuse, including prescription or recreational drugs; the presence of other liver diseases such as acute or chronic hepatitis C, acute or chronic active hepatitis B, Wilson's, autoimmune, alpha-1-antitrypsin and hemochromatosis or HIV infection; renal insufficiency; symptomatic coronary; peripheral or neurovascular disease; symptomatic heart failure or advanced respiratory disease requiring oxygen therapy; a history of cerebral or retinal hemorrhage or other bleeding diathesis.

### 3. Key Criteria for Measuring Baseline and Post Treatment Values:

Patients participating in this study are evaluated for one or more of the following criteria.
*a) Primary Long-Term Efficacy Outcome Measure*
   ● Histology at treatment month 12.5 to evaluate the NAS score, as a comparison to the baseline score measured pre-treatment. (NAS)
*b) Primary Short-Term Efficacy Outcome Measure*
   ● Change from baseline in ALT levels at Month 3 and Month 6 of treatment.
*c) Secondary Efficacy Outcome Measures*
   ● Overall NAS score
   ● Feature scores including fibrosis, ballooning degeneration, inflammation and steatosis
   ● Liver function tests (AST, alkaline phosphataise, bilirubin, GGT, Albumin)
   ● Cholesterol (incuding HDL and LDL)
   ● Triglycerides
   ● Fatty acid assay
   ● Ferritin
   ● Thioredoxin
   ● Pro-inflammatory cytokines (TNF-α, sTNF-R1, sTNF-R2, Hs-CRP, CTGF, sCD40)
   ● Insulin sensitivity (HOMA-IR)
   ● HbA1c
   ● Glucose
   ● Leptin, Serum adiponectin and complement factor D
   ● CK18 fragment and Serum HMGB1
   ● Soluble Fas antigen
   ● Hyaluronic acid
   ● Type IV collagen (7S domain)
   ● Procollagen III peptide
*d) Safety Outcome Measures*
   ● Adverse Events
   ● Hematology/biochemistry/urinalysis
   ● ECG (including QT/QTc measurement)
*e) Pharmacokinetic Outcome Measures*
   ● EPA, DPA and DHA
   ● Day 1
      ○ On Day 1, samples for plasma concentration are obtained at predose and 0.5, 1, 2, 4, 5 and 6 hours after Dose #1 and Dose #3; after Dose #2, samples are obtained at 2, 4, 5 and 6 hours post-dose. After Dose #3, samples are also obtained at 8 and 12 hours post-dose (20 and 24 hours after Dose #1 [prior to the morning dose on Day 2])
      o Cₘₐₓ (Dose #1 and Dose #2s) and Cₘₐₓ, Tₘₐₓ, T_{1/2}, AUG₀₋ₜ after third Dose are derived from plasma concentrations
   ○ Days 29, 85, 169 and 365 (Visits 3, 5, 7 and 9)
      o A single sample is obtained prior to the morning dose (trough) on Visits 3, 5, 7 and 9.
      ○ Css is determined from plasma concentrations

### 4. Concomitant and Medications:

Particular medications were prohibited or permitted during the study.

The following medications can be prohibited during treatment:
● Omega-3-acid ethyl esters and omega-3-PUFA containing supplements > 200 mg per day
● Vitamin E > 60 IU per day
● Thiazolidinediones (e.g. pioglitazone, rosiglitazone)

The following medications may be used during the study according to the specified restrictions:
● Subjects may continue prescription or over-the-counter medications or herbal remedies such as HMG-CoA reductase inhibitors (stains), fibrates, probucol, ezetimibe, ursodiol (UDCA), taurine, betaine, N-acetylcysteine,s-adenosylmethionine (SAM-e), milk thistle, anti-TNF therapies, or probiotics
● Subjects may continue the following anti-diabetic medications: biguanides (metformin), insulin, sulfonylureas, alpha-glucosidase inhibitors (acarbose), dipeptidyl-peptidase 4 inhibitors (sitagliptin, saxagliptin), and phenylalanine derivatives (nateglinide, repaglinide)
● Subjects may continue receiving anti-platelet therapy and anti-thrombotic agents (e.g. warfarin, ASA, and clopidogrel) after study commencement; should be monitored closely during the study for bleeding problems.

### 5. Treatment

210 patients are enrolled in the study and randomly divided into 3 equally sized groups. One of the groups receives a placebo (Placebo group) and to the other two groups (1800 mg per day group and 2700 mg per day group) EPA-E is administered three times per day, the total daily dose of EPA-E being 1800 mg or 2700 mg per day, divided into dosage amounts of 600 mg or 900 mg, respectively.

The treatment is continued for 12 months.

The patients are periodically evaluated for the selected criteria, at month 1, month 3, month 6 and month 12 of treatment.

After 12 months of treatment, patients are evaluated for the criteria noted above, including liver biopsy, NAS score, steatosis, lobular inflammation, ballooning and fibrosis stage, and one or more of the other criteria listed above in Table 1.

## Claims

1. Ethyl eicosapentanoate for use in the treatment or alleviation of symptoms of non-alcoholic steatohepatitis,
wherein said ethyl eicosapentanoate is administered in a dose of between 300 and 4000 mg per day.

2. Ethyl eicosapentanoate for use according to claim 1,
wherein said ethyl eicosapentanoate is administered in a dose of between 900 and 3600 mg per day.

3. Ethyl eicosapentanoate for use according to claim 1 or 2, wherein said dose is between 1800 and 2700 mg per day.

4. Ethyl eicosapentanoate for use according to any of claims 1 to 3, wherein said ethyl eicosapentanoate is administered once to three times per day in dosage amounts of 600 mg or 900 mg.

5. Ethyl eicosapentanoate for use according to any of claims 1 to 4, wherein said ethyl eicosapentanoate is administered for a period of between 3 and 24 months.

6. Ethyl eicosapentanoate for use according to claim 5, wherein said ethyl eicosapentanoate is administered for a period of between 6 and 12 months.

7. A pharmaceutical composition for use in the treatment or alleviation of symptoms of non-alcoholic steatohepatitis,
comprising ethyl eicosapentanoate as an active ingredient, wherein said ethyl eicosapentanoate is administered in a dose of between 300 and 4000 mg per day.

8. The pharmaceutical composition for use according to claim 7,
wherein said ethyl eicosapentanoate is administered in a dose of between 900 and 3600 mg per day.

9. The composition for use according to claim 7 or 8, wherein said composition further comprises one or more fatty acid.

10. The composition for use according to claim 9, wherein said fatty acid is Docosahexaenoic ethyl ester.

11. The composition for use according to claim 9 or 10, wherein the weight ratio between ethyl eicosapentanoate and the fatty acid is 0.8 or more.

12. The composition for use according to any of claims 9 to 11, wherein the daily dose in terms of total content of ethyl eicosapentanoate and said fatty acid is 0.3 to 10.0 g/day.
